(19) Europäisches Patentamt
European Patent Office
Office européen des brevets

(11) EP 3 092 069 B1

(12) FASCICULE DE BREVET EUROPEEN

(45) Date de publication et mention de la délivrance du brevet:
**02.08.2017 Bulletin 2017/31**

(21) Numéro de dépôt: **14827800.5**

(22) Date de dépôt: **04.12.2014**

(51) Int Cl.:
***B01J 13/14*** *(2006.01)* ***B01J 13/22*** *(2006.01)*
***C11D 3/50*** *(2006.01)*

(86) Numéro de dépôt international:
**PCT/FR2014/053165**

(87) Numéro de publication internationale:
**WO 2015/104469 (16.07.2015 Gazette 2015/28)**

# (54) PROCÉDÉ DE FABRICATION DE MICROCAPSULES À DOUBLE PAROI, MICROCAPSULES PRÉPARÉES PAR CE PROCÉDÉ ET LEUR UTILISATION

VERFAHREN ZUR HERSTELLUNG DOPPELWANDIGER MIKROKAPSELN, NACH DIESEM VERFAHREN HERGESTELLTE MIKROKAPSELN UND VERWENDUNG DAVON

PROCESS FOR MANUFACTURING DOUBLE-WALLED MICROCAPSULES, MICROCAPSULES PREPARED BY THIS PROCESS AND THE USE THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.01.2014 FR 1450166**

(43) Date de publication de la demande:
**16.11.2016 Bulletin 2016/46**

(73) Titulaire: **Microcapsules Technologies**
**45390 Puiseaux (FR)**

(72) Inventeur: **HABAR, Gérard**
**F-77780 Bourron-Marlotte (FR)**

(74) Mandataire: **Le Cloirec, Claudine et al**
**Ipsilon**
**3, rue Edouard Nignon**
**44300 Nantes (FR)**

(56) Documents cités:
**WO-A1-2008/009216 FR-A1- 2 937 248**
**US-A1- 2013 109 565**

- **DATABASE WPI Week 201202 Thomson Scientific, London, GB; AN 2011-H90576 XP002729124, & CN 102 079 970 A (TIANJIN DEHAO NEW MATERIALS CO LTD) 1 juin 2011 (2011-06-01)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un procédé de fabrication de microcapsules à réservoir, les microcapsules ainsi réalisées et leur utilisation dans des formulations telles que des lessives.

Les microcapsules appelées microcapsules à réservoir (ou encore dénommées microcapsules core/shell) sont des microcapsules du type renfermant un principe actif dans une enveloppe à base de polymère.

Les procédés de fabrication de ces microcapsules, et donc d'incorporation du principe actif dans un polymère comprennent les étapes consistant à:

- disperser au moins un principe actif lipophile, respectivement hydrophile, dans une phase continue aqueuse ou une phase continue organique, de manière à former une évulsion ou une dispersion de gouttelettes respectivement de type huile dans l'eau ou eau dans huile,

- polymériser in situ un précurseur du polymère à la périphérie desdites gouttelettes pour former la paroi de l'enveloppe des microcapsules, en enfermant le principe actif.

## ETAT DE LA TECHNIQUE :

La plupart des procédés d'encapsulation mis en oeuvre industriellement de nos jours concernent des procédés impliquant la polymérisation de monomères ou de pré-polymères organiques aminés en présence d'aldéhyde(s). Les procédés tels que ceux décrits notamment dans les brevets US 4,406,816 de BASF, US 4,824,823 de KOEHLER, ou US 4,396,670 de WIGGINS TAEPE utilisent des résines mélamine/formol qui conduisent à des microcapsules qui sont étanches mais ne résistent pas en présence de détergents.

Un autre inconvénient de ces microcapsules est leur teneur élevée en formaldéhyde résiduel, malgré les divers traitements mis au point pour en diminuer le taux, tels que les traitements explicités dans les documents EP 1797947 ou US 7,968,510.

Pour abaisser la teneur en formaldéhyde résiduel, divers copolymères ont été présentés, tels ceux décrits dans la demande de brevet US 2010/0009893.

Enfin des résines aminées fabriquées sans formaldéhyde destinées à l'encapsulation ont été décrites, comme dans les demandes de brevets US 2010/0247941 ou WO/2011/161618, sans que la tenue au tensio-actifs soit améliorée.

D'autres procédés mettant en oeuvre des monomères de type silicates ou silicone ont été proposés, notamment dans le brevet FR 2937248, pour réaliser l'enveloppe des microcapsules. Cependant ces microcapsules sont difficiles et coûteuses à fabriquer et n'offrent pas une tenue satisfaisante aux tensio-actifs.

L'inconvénient majeur de l'ensemble de ces microcapsules de l'art antérieur est un défaut d'étanchéité de l'enveloppe polymère, notamment lorsque les microcapsules sont mises en présence d'agents tensio-actifs, notamment dans des formulations telles que les lessives, les shampoings, ou les détergents par exemple. En effet les tensio-actifs sont connus pour être des "microcapsules killers" car ils extraient les constituants qui sont à l'intérieur des microcapsules et les libèrent.

Des procédés d'encapsulation multicouches ont été mis au point à partir de monomères ou de pré-polymères organiques aminés, pour renforcer l'étanchéité de l'enveloppe, mais ces procédés doivent s'effectuer en plusieurs étapes, et ne résolvent notamment pas la question de l'étanchéité en présence de tensio-actifs.

Un premier but de l'invention est de proposer un procédé de fabrication de microcapsules à réservoir permettant d'incorporer un actif, dans une enveloppe polymère présentant une meilleure étanchéité aux tensio-actifs que les microcapsules de l'art antérieur.

Un autre but de l'invention est de proposer un procédé de fabrication de microcapsules à réservoir présentant une faible teneur en aldéhyde(s) résiduels.

Un autre but de l'invention est de proposer une procédé d'incorporation de principe actif de type parfum dans une suspension ou dispersion de microcapsules à réservoir destinées à être utilisées dans des formulations renfermant des agents tensio-actifs.

## Résumé de l'invention :

Ces buts sont atteints par le procédé selon la présente invention qui concerne un procédé de fabrication de microcapsules à réservoir (en suspension aqueuse), du type renfermant un principe actif dans une enveloppe à base de polymère comprenant les étapes consistant à:

(i) disperser au moins un principe actif lipophile dans une phase continue aqueuse, de manière à former une émulsion ou une dispersion de gouttelettes de type huile dans l'eau,

(ii) polymériser in situ au moins un précurseur du polymère à la périphérie desdites gouttelettes pour former la paroi de l'enveloppe des microcapsules, en enfermant le principe actif,

caractérisé en ce que
l'étape de polymérisation (ii) est précédée de l'introduction dans la phase lipophile d'un ou plusieurs composés A portant des groupes alcoxysilanes et l'introduction dans la phase aqueuse de monomères organiques aminés B comportant au moins un groupe choisi parmi les groupes mélamine, urée, glycoluril, benzoguanamine, ou dicyandiamide et d'un ou plusieurs aldéhydes, et/ou de leurs pré-polymères,
les composés A et B étant ensuite, de préférence simultanément, respectivement hydrolysés et condensés en milieu acide en un polymère silicone et un polymère organique aminé, liées entre eux par des liaisons polaires, hydrogène ou covalentes, composant la paroi de l'enveloppe des microcapsules.

Les polymères de silicone et les polymères organiques aminés sont réputés incompatibles, et aucun procédé de l'art antérieur d'encapsulation multicouche ne combine ces deux types de polymères. De manière surprenante le procédé selon la présente invention permet non seulement de mettre en oeuvre ensemble des polymères incompatibles, mais en plus de les polymériser simultanément ou quasi simultanément si l'on introduit les composés B avec un léger retard pour donner un peu d'avance à la formation du polymère silicone. Ce procédé, simple, permet ainsi d'encapsuler aisément un actif lipophile dans une enveloppe composite à double paroi : une paroi polymère silicone entourée d'une paroi en polymère organique aminé.

## Description de l'invention

De telles enveloppes d'encapsulation combinent alors les propriétés des membranes de silicone à celle des membranes organiques et conduisent à un effet barrière bien plus important que l'effet procuré par chacune d'elles, effet barrière qui prend toute son importance lorsque les microcapsules sont utilisées notamment en présence d'agents chimiques tels que les tensio-actifs.

L'intérêt du procédé selon la présente invention est également de diminuer la teneur finale en aldéhyde du fait de la constitution de l'enveloppe polymère, à savoir que le polymère aminé ne représente qu'une partie de l'enveloppe des microcapsules.

En effet on remarque que le taux de formaldéhyde résiduel, comparé à celui d'une encapsulation classique par polymérisation de résine mélamine/formaldéhyde ou urée/formaldéhyde, est considérablement réduit du fait même de la quantité moindre de résine mélamine formaldéhyde ou urée formaldéhyde utilisée, puisqu'une partie est remplacée par un polymère de silicone, mais également parce que la résine de silicone ne retient pas le formaldéhyde. Ceci est un grand avantage de ce type de microcapsules dans un grand nombre d'applications, comparé aux microcapsules de mélamine d'utilisation courante.

De plus, l'utilisation de deux polymères différents pour réaliser ladite enveloppe des microcapsules permet, en faisant varier leurs proportions respectives, d'élaborer des microcapsules sur mesure, adaptées le plus possible à leur environnement final, et également optionnellement d'assurer à l'ensemble de la structure de bonnes performances mécaniques par des réactions chimiques liant les deux types de polymères.

Le procédé de l'invention présente également l'avantage de la fabrication de microcapsules "à réservoir" à partir de monomères bon marché largement disponibles que sont les précurseurs du polymère de type silicone, tout en mettant en oeuvre une technique d'encapsulation générale par ailleurs déjà connue de l'homme de l'art pour le polymère organique aminé.

De préférence, les deux polymères silicone et organique aminé sont formés simultanément par catalyse acide à un pH compris entre 2 et 6.

En effet, l'ajout d'un acide catalyse non seulement la polymérisation de la résine aminée, mais également l'hydrolyse des groupes SiOR en SiOH ainsi que leur subséquente réaction en 2 SiOH $\rightarrow$ Si-O-Si + $H_2O$. Etonnamment, et c'est là une des clés de cette invention, il a été constaté que les acides habituellement utilisés pour la polymérisation des résines aminées tels que l'acide formique, chlorhydrique, sulfurique, nitrique, sulfonique étaient également susceptibles de polymériser simultanément le polymère de silicone dans les conditions de pH entre 2 et 6 et à une température adéquate de façon à permettre l'hydrolyse des groupes Si-O-R avant que la membrane organique ne devienne trop imperméable à l'eau.

De manière avantageuse, les deux polymères silicone et organique aminé sont formés simultanément par catalyse acide à un pH situé entre 3 et 5, par ajout dans l'émulsion ou dispersion huile dans l'eau d'au moins un acide comprenant l'acide nitrique. Cet acide est en effet un très bon catalyseur de l'hydrolyse et de la polymérisation subséquente des groupes siloxane, et de plus il s'avère compatible avec la polymérisation des résines aminées.

Selon un mode de réalisation préféré de l'invention, le pré-polymère organique aminé est une résine mélamine-formaldéhyde et/ou urée-formaldéhyde. Ce type de pré-polymère a l'avantage d'être facilement disponible industriellement et bon marché, et sa polymérisation bien décrite et connue de l'homme de l'art. Par contre la présence de formaldéhyde peut s'avérer rédhibitoire dans beaucoup d'applications. Dans ces applications on préfère alors, pour fabriquer le polymère organique aminé, utiliser un aldéhyde autre que le formaldéhyde, à savoir un aldéhyde avantageusement choisi parmi l'acétaldéhyde, le glyoxal, le glutaraldéhyde, ou un mélange de ceux-ci, et/ou un ou plusieurs acétals de ces aldéhydes.

La mélamine et/ou l'urée peuvent également préférentiellement être utilisés avec ces aldéhydes autres que le formaldéhyde, mais il est également possible pour des raisons de cinétique de réaction ou de performance finale de les remplacer totalement ou partiellement par une autre molécule aminée connue telles que le glycoluril et/ou la benzoguanamine et/ou le dicyandiamide par exemple, pour réagir avec les aldéhydes et conduire à des polymères possédant de bonnes performances.

La résine aminée sans formaldéhyde peut être également préparée à partir des monoacétals ou des diacétals de ces mêmes aldéhydes, ou du mélange d'aldéhydes et d'acétals, elle peut être estérifiée, réticulée par des polymères portant des groupes hydroxyle, mercapto, acide carboxylique, amine, amide qu'ils soient aromatiques tels les phénols et leurs dérivés porteurs de groupes acide carboxylique ou sulfonique, ou aliphatiques tels les oses et les dérivés cellulosiques.

La substitution des résines mélamine/formaldéhyde ou urée/formaldéhyde par une résine sans formaldéhyde est plus simple à réaliser dans le procédé selon la présente invention que dans le cas d'une polymérisation in situ d'une résine aminée seule du fait que la structure de la microcapsule repose en partie sur le polymère de type silicone et que le polymère aminé peut ne représenter qu'une faible part de la paroi de la microcapsule. Dans ces conditions il n'est pas nécessaire de réaliser un pré-polymère avec les aldéhydes et les molécules aminées comme c'est le cas dans les techniques décrites précédemment, mais il est possible de synthétiser le polymère organique aminé directement dans la phase aqueuse en introduisant séparément les aldéhydes ou leur acétals et les molécules aminées mentionnées précédemment telles la mélamine, l'urée etc.., ce qui est un avantage économique très intéressant car il supprime une étape dans la mise en oeuvre du procédé.

En variante le polymère organique aminé peut être copolymérisé avec des monomères hydroxylés aliphatiques ou aromatiques et/ou des aldéhydes aromatiques, ce qui peut s'avérer judicieux pour augmenter encore l'étanchéité des microcapsules et leur tenue vis-à-vis des tensio-actifs.

De préférence le ou les composés A portant des fonctions alcoxysilanes est/sont choisi(s) parmi les composés de formule (I) ou (II) ci-après :

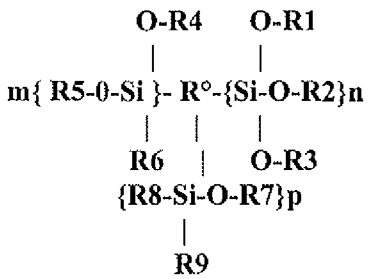

et/ou

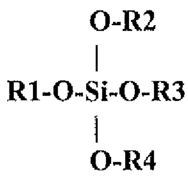

(I) (II)

dans lesquels R1, R2, R3, R4, R5, R6, R7, R8, R9 sont des radicaux alkyle, linéaires ou cycliques, substitués ou non,

R° est une molécule organique et/ou silicone,

les groupes entre {} étant reliés à R° par un atome de silicium et sont présents m, n, ou p fois, et m, n, p pouvant être nuls individuellement, mais la somme m+n+p étant au moins égale à 1.

A titre d'exemples les monomères ou pré-polymères (I) utilisables pour synthétiser le polymère de silicone peuvent être choisis dans la liste non limitative d'après :

- des monomères ou pré-polymères de trialkoxysilane R-Si $(OR')_3$ dans lequel R représente un radical alkyle à 1 à 20 atomes de carbone, substitué ou non tels par exemple les radicaux méthyle, éthyle, n-propyle, isopropyle, 1-n-butyle, 2-n-butyle, isobutyle, tert-butyle, n-pentyle, isopentyle, neopentyle, tert-pentyle, hexyle tel que le n-hexyle, heptyle tel que le n-heptyle, octyle tel que le n-octyle ou l'isooctyle, 2,2,4-triméthylpentyle, nonyle, décyle, dodécyle, ou octadécyle ;

- R peut être également un radical cyclique, cycloalkyle tel que le cyclopentyle, le cyclohexyle, le cycloheptyle et le

méthylcyclohexyl, également un radical aryle tel que le phényle, le naphthyle, l'anthryle et le phénanthryle, ou un radical alkaryle tel que les o-, m- ou p-tolyle, xylyle et éthylphenyle, ou encore un radical aralkyle tels que les benzyle, alpha.-et beta.-phényléthyle ;

- R peut également être halogéné tel que par exemple les 3,3,3-trifluoro-n-propyle, 2,2,2,2',2',2'-hexafluoroisopropyle, heptafluoroisopropyle, o-, m- ou p-chlorophényle ;

- R peut être un radical insaturé tel que les radicaux vinyle, 5-hexènyle, 2,4-divinylcyclohexyléthyle, 2-propènyle, allyle, 3-butènyle, 4-pentènyle, éthynyle, propargyle and 2-propynyle ;

- R peut aussi porter des groupes réactifs susceptibles de réagir avec le polymère organique, tel les groupes NH, OH , COOH, époxyde, urée ou SH.

Sont cités ci-après à titre d'exemples non limitatifs quelques monomères intéressants parmi lesquels :

- les silanes chlorés tel le chloropropyl méthyl diméthoxysilane,

- les silanes porteurs de groupes isocyanates tel l'isocyanate propyltriéthoxysilane,

- les époxydes tel le glycidoxy propyltriméthoxy ou triéthoxy silane, le glycidoxy propylméthyldiéthoxysilane, (3,4-époxycyclohexyl)éthyltriméthoxy ou triéthoxy silane,

- les silanes acryliques: l'acryloxy propyl triméthoxysilane, le méthacryloxy propyl triméthoxysilane, le gamma.-méthacryloxy propylméthyl diméthoxy- ou diéthoxysilane,

- les silanes porteurs de groupes thiols ou d'atomes de soufre : le mercaptopropylmethyl dimethoxysilane, le Bis-{3-(triéthoxysilyl) propyl} polysulfure, le Bis-{3-(triéthoxysilyl) propyl} disulfure, le 3-octanoylthio-1-propyl triéthoxysilane,

- les silanes aminés : 3-aminopropyltriéthoxy- ou méthoxy- silane, N-(nbutyl)-3-aminopropyl triméthoxy- ou éthoxysilane, N-aminoéthyl-3-aminopropylméthyl diméthoxysilane, N-aminoéthyl-3-aminopropyl triméthoxy- ou triéthoxysilane, 3-aminopropylméthyl diéthoxysilane, N-phénylaminopropyl triméthoxysilane, 2-aminoéthylaminopropyl triméthoxysilane, 2-aminoéthylaminopropylméthyl diméthoxysilane, anilinopropyl triméthoxysilane, gamma.-[N-(.beta.-aminoéthyl)amino] propylméthyl diméthoxysilane, 4-amino-3,3-diméthylbutyl triméthoxysilane, 4-amino-3,3-diméthylbutylméthyl diméthoxysilane, Bis-{gamma-(triméthoxysilyl) propyl}amine, N-éthyl-gamma-aminoisobutyl triméthoxysilane, 3-ureidopropyl triéthoxysilane, hexaméthyldisilazane, alkylèneoxyde triméthoxysilane, Tris-{3-(triméthoxysilyl) propyl} isocyanurate, Bis(triéthoxysilyl)éthane.

Le choix est très large ce qui rend cette technologie très malléable et polyvalente et permet de fabriquer des microcapsules « sur mesure », qui peuvent être adaptées à leur utilisation finale.

R' est en général un radical alkyle, préférentiellement à chaine courte tels que les radicaux méthyle et éthyle qui présentent des vitesses de réaction plus élevées, ou encore un radical porteur d'un atome d'oxygène, par exemple choisi parmi les radicaux méthoxyéthyle, éthoxyéthyle, acétoxy ou oxymino, ou un radical alkyle porteur d'un atome d'halogène, le chlore étant préféré.

Des monomères tels que les monoalkoxysilane ou dialkoxysilane destinés à diminuer le degré de réticulation et ainsi assouplir le polymère de silicone sont également intéressants.

Il est bien entendu possible, et intéressant en fonction des utilisations finales envisagées des microcapsules, de mettre en oeuvre dans le procédé selon la présente invention, des monomères plus complexes que ceux précédemment cités, tels :

- les tris alkoxy isocyanurates, les bis alkoxy silanes,

- des adducts tels ceux décrits dans le brevet français FR 2.913.887, ceux obtenus en faisant réagir entre eux des silanes porteurs de groupes réactifs tel le glymo (3-glycidoxypropyl) triméthoxysilane) avec un silane aminé comme le 3-aminopropyltriéthoxy silane, le glymo avec un mercaptosilane, un mercapto silane avec un l'isocyanato propyl triéthoxysilane, l'isocyanato propyl triéthoxysilane avec le 3-aminopropyltriéthoxy silane, etc... Ces adducts présentent l'intérêt d'offrir un rapport poids de polymère/poids de monomère plus élevé que les siloxanes classiques, ce qui diminue la quantité d'alcool libéré et d'eau consommée par unité de poids de polymère silicone formé. Ceci est

un avantage compte tenu du fait que l'hydrolyse et le départ d'alcool qui résulte de la polymérisation du silicone sont gênés par le polymère aminé en formation autour des microcapsules. Ainsi en diminuant la quantité de ces produits migrant à l'interface huile/eau on augmente l'efficacité des réactions de polymérisation du polymère de silicone et la vitesse de la réaction,

- des composés obtenus par réaction d'une molécule hydrocarbonée portant des groupes réactifs réagissant avec les groupes réactifs présents dans la chaine carbonée de l'organosiloxane comme par exemple ceux obtenus par réaction d'un époxyde organique avec un silane aminé, ou ceux obtenus par réaction d'un polyisocyanate organique avec du MTMO (3-mercaptopropyl triméthoxysilane), ou ceux obtenus par réaction d'un polythiol organique avec du glymo. Cette dernière catégorie de molécule présente l'avantage d'être plus compatible avec le polymère organique aminé.

En ce qui concerne la nature de la molécule de type silicate (II) que l'on peut utiliser seule ou alliée à la structure silicone, les monomères et pré-polymères de type ester siliciques $Si(OR')_4$, (dans lesquels R' possède la même signification que les groupes décrits précédemment) donnent également de bons résultats, en particulier les polysilicates de méthyle et/ou d'éthyle qui sont très courants et bon marché et ont la particularité d'être très lipophiles et donc de ne pas diffuser dans la phase aqueuse même quand ils sont complètement hydrolysés.

Selon un mode de réalisation avantageux du procédé de la présente invention le composé de formule (II) est donc choisi parmi le polysilicate de méthyle, le polysilicate d'éthyle ou un mélange de ceux-ci.

La liste ci-dessus n'est pas limitative : elle peut aussi peut englober les oligomères de ces produits. Un des critères importants est que le composé portant les groupes alkoxysilane reste liquide et au moins partiellement soluble, ou dispersible, dans la phase lipophile, et de préférence insoluble dans la phase aqueuse.

On voit donc que les possibilités sont innombrables et permettent de régler finement les propriétés de la membrane de la microcapsule, notamment sa perméabilité et sa tenue aux agents chimiques extérieurs, tels que les composés tensio-actifs.

Dans un des modes de réalisation le plus simple, on introduit sous agitation dans la phase lipophile, renfermant l'actif, phase qui deviendra la phase interne des microcapsules après dispersion dans l'eau, les polymères hydrolysables porteurs de groupes Si-O-R, à savoir du polysilicate d'éthyle et optionnellement un mélange de méthyltriéthoxysilane et de silanes possédant un ou des groupes susceptibles de réagir avec la résine organique aminée utilisée. Dans le cas courant de l'utilisation dans la phase aqueuse d'une résine mélamine-formaldéhyde ou urée-formaldéhyde une partie des silanes portera utilement sur leur partie non hydrolysable des groupes tels que OH, SH (MTMO par exemple) époxyde (glymo par exemple) ou urée (uréido propyl triméthoxysilane par exemple). On agite jusque à ce que le milieu réactionnel soit homogène et transparent. Le type de silane ou de silicate et leur proportion est évidemment à choisir de façon que ces produits soient suffisamment solubles dans la phase lipophile, de façon à obtenir dans la mesure du possible, mais cela n'est pas impératif, une solution transparente favorable à l'édification des parois de la microcapsule.

Cette phase lipophile ainsi constituée, étant en général destinée à devenir la phase interne des microcapsules, est dispersée dans de l'eau contenant les polymères et colloïdes protecteurs nécessaires habituels permettant l'édification du polymère de résine aminée. Le cas échéant on peut introduire dans la phase aqueuse une partie des silicates ou silane, entre autre ceux susceptibles de réagir sur la résine aminée. Il est à noter que de toute façon la résine aminée réagit au moins partiellement sur les groupes silanol si les conditions sont appropriées.

Ici encore, d'une façon inattendue et étonnamment simple, l'introduction d'acide provoque la dépose du polymère par polymérisation du pré-polymère amine-aldéhyde, en même temps qu'a lieu la polymérisation simultanée du silicone à l'interface au contact de l'eau. Tout se passe au départ comme si le polymère aminé en formation jouait le rôle de colloïde protecteur vis-à-vis de la formation des microcapsules à paroi de silicone, ce qui est particulièrement inattendu.

Les opérations suivantes, telles que refroidissement, remontée en pH, traitement visant à diminuer le taux de formaldéhyde résiduel quand il est présent, introduction de conservateurs, épaississement etc...sont classiques donc bien connues de l'homme de l'art.

Le polymère dit silicone et le polymère organique forment alors la double paroi composite de l'enveloppe de la microcapsule dans des proportions très variables. Les effets intéressants obtenus notamment sur le taux éventuel de formaldéhyde résiduel, sur l'étanchéité et sur la tenue aux tensio-actifs, se situant lorsque le polymère de silicone représente avantageusement de 5 % à 95 %, de préférence de 10 à 90%, de préférence encore de 15 à 85 %, du poids total des polymères formant la paroi de la microcapsule.

Autour d'un rapport pondéral polymère silicone/polymère aminé voisin de 10/90 on obtient des microcapsules de polymère aminé améliorées par une mince couche de silicone intérieure, et autour d'un rapport pondéral polymère silicone/polymère aminé voisin de 90/10 des microcapsules de silicone protégées par une mince couche de polymère aminé. Entre ces deux extrêmes peuvent être fabriquées toute une gamme de microcapsules adaptées à leur milieu d'utilisation finale.

Comme bien connu de l'homme de l'art, la formation de l'émulsion et le maintien de son intégrité au cours de l'encapsulation est favorisé par l'introduction d'un polymère hydrosoluble dans la phase continue aqueuse, dit colloïde protecteur.

Sont cités à titre non limitatif de colloïde protecteur les dérivés de cellulose tels que les dérivés hydroxyéthylcellulose, carboxyéthylcellulose et méthylcellulose, les polyvinylpyrrolidone et les copolymères de polyvinylpyrrolidone, les alcools polyviniliques plus ou moins hydrolysés ainsi que leur copolymères, les polymères d'origine naturelle comme la gélatine, la gomme de xanthane ou la gomme arabique, les alginates, pectines, amidons et dérivés, la caséine ainsi que des polymères ionisés tels que les polymères et copolymères d'acide acrylique ou méthacryliques, les polymères porteurs de groupements acide sulfoniques ou carboxyliques ou de leur anhydrides ou de groupements amine cationisés.

Comme indiqué précédemment la polymérisation simultanée du silicone et de la résine aminée est catalysée à pH compris entre 2 et 6, préférentiellement entre 3 et 5. Divers catalyseurs métalliques ou organométalliques peuvent être utilisés pour compléter la réaction de polymérisation. Peuvent être cités, de manière non limitative, les composés renfermant de l'étain tels le dilaurate ou le diacétate de dibutylétain, l'octoate d'étain, les sels minéraux d'étain, les composés de platine, de zinc, de zirconium, d'aluminium, de titane dont les titanates et les fluorures.

Ces composés permettent d'abaisser de façon significative le taux d'alkoxy ou de silanol résiduels, ce qui peut être pour certaines applications très intéressant et permet d'éviter les réactions de réversion.

La température de départ dépend de l'ingrédient à encapsuler, mais est surtout imposée par la polymérisation du polymère organique. Elle est envisageable entre 15°C et 55 °C, de préférence entre 20°C et 50°C.

Pour terminer la polymérisation la température est souvent graduellement élevée jusqu'à une valeur située entre 60 et 90°C, valeurs qui ne sont qu'indicatives car les plages optimum de température dépendent beaucoup de l'actif qui est encapsulé.

Le départ de l'alcool formé lors de l'hydrolyse des groupes alkoxysilane est favorisé par une température finale élevée, ceci est d'autant plus nécessaire que le polymère organique aminé fait barrière et donc freine ce départ, ce qui allonge le maintien à haute température quand on compare cette technologie à l'encapsulation simple par résine mélamine/formol par exemple. Les pertes de matières volatiles sont en général compensées si nécessaire par des apports en eau au cours de l'encapsulation.

L'opération terminée on ramène de préférence le milieu réactionnel à la température ambiante où les microcapsules peuvent être conservées et utilisées en l'état, dans la suspension aqueuse. En fait, les milieux et conditions d'utilisation des microcapsules font qu'il est souvent préférable de remonter le pH à des valeurs situées entre 5 et 8 environ avec de la soude, de la potasse, des amines ou tout autre moyen connu de l'homme de l'art.

Les microcapsules obtenues par le procédé selon l'invention peuvent être ensuite séchées, séparément ou avec d'autres ingrédients, dans une tour d'atomisation, sur lit fluidisé, par cryoséchage, ou par tout autre moyen pour être utilisées dans des compositions sèches.

Les actifs lipophiles pouvant être encapsulés selon l'invention décrite sont très nombreux, la seule limitation étant qu'ils supportent les conditions de température et de pH imposées pendant les opérations d'encapsulation et soient compatibles avec les alkoxysilanes ou les silicates.

Les composés d'intérêt sont ceux habituellement commercialisés encapsulés par d'autres techniques de l'art antérieur.

Nous citerons parmi les actifs intéressants à encapsuler notamment les acides et alcools gras, les solvants organiques, les hydrocarbures, les esters, les fluides et gommes silicone, les huiles végétales et extraits végétaux, en particulier les produits connus pour leur intérêt cosmétique comme les huiles végétales et les huiles essentielles, les colorants réactifs ou non réactifs, ainsi que les dispersions de pigments, les filtres UV, les vitamines et molécules médicalement actives, les parfums et arômes, les insecticides et répulsifs, les catalyseurs, les matériaux à changement de phase, les composés phénoliques, des adhésifs et réactifs chimiques, sans que cette liste soit limitative.

La présente invention concerne également les microcapsules préparées au moyen du procédé décrit précédemment, comprenant un polymère silicone et un polymère organique aminé, liés entre eux par des liaisons polaires, hydrogène ou covalentes, composant la paroi de l'enveloppe des microcapsules, utilisées dans des formulations contenant des tensio-actifs, plus particulièrement les microcapsules renfermant en tant que principe actif une molécule odorante, tel qu'un parfum.

Les microcapsules selon l'invention peuvent être semi-perméables ou étanches. Ceci peut être réalisé par l'homme du métier en jouant sur les conditions de polymérisation de la paroi ainsi que sur les caractéristiques dimensionnelles des microcapsules tel le diamètre et l'épaisseur de la paroi.

Le ratio pondéral paroi/contenant des microcapsules peut varier dans de grandes proportions, par exemple entre 5 et 50%, préférentiellement entre 10 et 30%, préférentiellement encore entre 15 et 20 %. Si ce ratio est trop faible, la paroi est fine, poreuse et présente une fragilité mécanique et chimique. Si le ratio correspondant à la paroi est trop important (par exemple au-dessus de 50 %) les microcapsules sont trop solides, et ne peuvent plus libérer l'actif. Elles sont en outre trop chères car nécessitant une quantité importante de polymères.

La dispersion finale des microcapsules dans l'eau du milieu réactionnel contient en général de 30 à 50% d'actif

contenu à l'intérieur des microcapsules, elle peut être diluée ou concentrée par les moyens habituels, ou encore séchée et disponible sous la forme d'une poudre pulvérulente (dans ce cas la concentration d'actif peut atteindre 80 % environ).

Les microcapsules selon la présente invention peuvent être utilisées dans toutes les applications où sont utilisées à ce jour les microcapsules fabriquées par les techniques de l'art antérieur : par exemple dans l'industrie cosmétique (notamment filtres UV, vitamines, huiles insaturées, actifs hydrophiles ou lipophiles de toute catégories, colorants, parfums), dans l'industrie du papier (autocopiant type NCR, papiers de sécurité, tissues tel que mouchoirs, lingettes, publicités parfumées par exemple), dans l'industrie textile (cosméto-textile, parfums, PCM), dans l'industrie du cuir, en pharmacie, en médecine, dans l'industrie vétérinaire, dans le domaine des adhésifs, peintures et enduits, ainsi que dans le domaine du bâtiment.

La présente invention concerne ainsi l'utilisation des microcapsules préparées au moyen du procédé décrit précédemment notamment dans des lessives liquides, des lessives en poudre ou des détergents ménagers et industriels, dans des adoucissants pour textiles ou encore dans des shampoings, des produits de conditionnement pour cheveux, des dentifrices, des savons liquides, des nettoyants corporels ou des lotions.

L'invention va maintenant être illustrée par les exemples non-limitatifs ci-après.

**Exemple 1 :** Microcapsules contenant un répulsif contre les moustiques

Dans un bécher de 800 $cm^3$ maintenu à 30°C on introduit sous agitation :

- 300 g d'eau du robinet
- 48,6 g de Lupasol PA 140 (BASF) (acrylamide : colloïde protecteur)
- 52,5 g d'une résine mélamine formol (Luracoll SD BASF)

L'agitateur est équipé d'une turbine défloculeuse d'un diamètre de 7 cm. La vitesse d'agitation est augmentée à 900 tr/min. Puis un mélange homogène de 216 g de citronnelle de chine et de 24 g de polysilicate d'éthyle (TES 40 Wacker) est émulsionné dans le mélange aqueux précédent.

On acidifie pour polymériser le tout par 11,1 g d'acide formique à 10% jusqu'à un pH d'environ 3,5.

La température est maintenue à 35°C pendant 2h, période durant laquelle la vitesse d'agitation est réglée à environ 1500 tr/mn de façon à obtenir un diamètre moyen de microcapsules de 6μm.

Après obtention de ce diamètre, on réduit la vitesse à 1200 tr/mn puis on porte la température à 80°C pendant 2h30 pour effectuer la polymérisation complète des deux couches. L'émulsion est alors refroidie à 30°C. Le formaldéhyde est neutralisé par introduction lente d'ammoniaque jusqu'à pH 9,0.

Résultats : comparées aux mêmes microcapsules réalisées sans polysilicate d'éthyle, ces microcapsules étalées sur un papier ont une odeur moins forte, montrant une meilleure étanchéité, et surtout un taux de formaldéhyde inférieur.

Exemple 2 : Microcapsules contenant un parfum mentholé

Dans un bécher de 500 $cm^3$ maintenu à 35°C on introduit sous agitation :

- 110 g d'eau du robinet
- 16 g de Lupasol PA 140 (BASF)
- 15 g d'une résine mélamine formol (Cymel 373 de Cytex)

L'agitateur est équipé d'une hélice à 5 pales droites d'un diamètre de 6 cm.

La vitesse d'agitation est augmentée jusqu'à 750 tr/min puis un mélange homogène de 86g de parfum mentholé, de 5,6 g de polysilicate d'éthyle (TES 40 Wacker), de 2,8 g de méthyltriéthoxysilane et de 1,2 g de MTMO (mercapto propyl triméthoxy silane) est émulsionné dans le mélange aqueux précédent.

On acidifie pour polymériser le tout par 4,4 g d'acide formique à 10%, le pH est alors de 3,5.

La température est maintenue à 35°C pendant 2h30 puis à 45°C pendant 1h, période durant laquelle la vitesse d'agitation est réglée à 1100 tr/min de façon à obtenir un diamètre moyen de particules de 15 μm, puis elle est diminuée à 900 tr/mn. La température du milieu réactionnel est ensuite portée à 80°C pendant 3h pour effectuer la polymérisation complète des 2 couches.

L'émulsion est alors refroidie à 30°C. Le formaldéhyde est neutralisé par introduction lente d'ammoniaque

jusqu'à pH 9,0.

Résultats : comparées aux mêmes microcapsules réalisées sans ajouts de composés silanes et silicate dans la phase lipophile renfermant le parfum, ces microcapsules étalées sur un papier ont une odeur moins forte, montrant une meilleure étanchéité, et surtout un taux de formaldéhyde inférieur.

**Exemple 3** : Microcapsules contenant un parfum

1) préparation du monomère de silicone A:

Dans un bécher est mélangé à l'abri de l'oxygène et de l'humidité (circulation d'azote sec) :

- 0,01 mole de ETTMP 700 (triméthylolpropane tri-3-mercapto-propionate éthoxylé 700) vendu par Bruno Bock, soit 7,08 g
- 0,025 mole de glymo (gamma-glycidoxy,triméthoxysilane), soit 5,9 g.

Le tout est mélangé et porté 10h à 60°C puis refroidi à température ambiante.

2) encapsulation

111 g de parfum Blue Wave d'expressions parfumées est mélangé de façon à obtenir une solution transparente à 6,0 g du monomère de silicone A préparé précédemment et à 14,8 g de polysilicate d'éthyle TES 40 de Wacker. Ce mélange constituera la phase interne des microcapsules.

Dans un bécher porté à 30°C sont alors introduits 130 g d'eau, 24 g de Lupasol PA140 de BASF (Colloïde protecteur) et 8,5 g de résine mélamine formol Beetle PT336 de BIP.

La phase interne préparée précédemment est introduite et émulsionnée sous agitation dans le mélange aqueux précédent, l'hélice inox de 6cm de diamètre étant portée à 1200 tr/min, on introduit alors 1,22 g d'acide nitrique à 20% pour obtenir un pH du milieu réactionnel de 4,15.

La température de 30°C est maintenue 2h, puis on chauffe 30 min à 40°C. On introduit alors 0,2 g d'acide chlorhydrique à 20% puis on laisse 1h à 40°C.

Durant ces étapes on abaisse la vitesse de l'agitateur à 800 tr/min dès que le diamètre de 12μm est atteint.

La température est alors portée à 80°C pendant 6h pour finir la polymérisation simultanée des deux couches de polymère. Le pH est ensuite remonté à 9,3 avec de l'ammoniaque pour abaisser le taux de formol résiduel.

Les microcapsules obtenues ont une durée de vie dans les adoucissants textiles et les lessives liquides supérieure à 6 mois. Le taux de formaldéhyde résiduel est de l'ordre de 100 ppm.

**Exemple 4 :** Microcapsules renfermant un parfum

1) préparation du monomère de silicone : idem exemple 3

2) Encapsulation :
111 g de parfum « Blue wave d'expressions parfumées » est mélangé de façon à obtenir une solution transparente à 5,0 g du monomère de silicone préparé précédemment et à 14,8 g de polysilicate d'éthyle TES 40 de Wacker. Ce mélange constituera la phase lipophile interne des microcapsules.

Dans un bécher porté à 30°C sont introduits 130 g d'eau, 20,2 g de Lupasol PA140 de BASF (Colloïde protecteur), 7,8 g de résine mélamine/formol Beetle PT336 de BIP et 1,75 g de téréphtalaldéhyde.

La phase lipophile préparée précédemment est introduite et émulsionnée sous agitation dans le mélange aqueux précédent, l'hélice inox de 6 cm de diamètre étant portée à 1200 tr/min, on introduit alors 1,15 g d'acide nitrique à 20% pour obtenir un pH du milieu réactionnel de 4,10.

La température de 30°C est maintenue 2h, puis l'ensemble est chauffé 30min à 40°C.

On introduit alors 0,8 g de xylitol en poudre et 1 g de résine mélamine/formol Beetle PT336 de BIP puis on laisse 1h30 à 40°C.

Durant ces étapes on abaisse la vitesse de l'agitateur à 800 tr/min dès que le diamètre de 12μm est atteint.

La température est alors portée à 80°C pendant 6h pour finir la polymérisation simultanée des deux couches de polymères. Puis le pH est remonté à 9,3 avec de l'ammoniaque pour abaisser le taux de formol résiduel.

Les microcapsules obtenues ont une durée de vie, dans les adoucissants textiles et les lessives liquides, supérieure à 10 mois. Le taux de formaldéhyde résiduel est de l'ordre de 90 ppm.

**Exemple 5 :** Microcapsules sans formaldéhyde renfermant un parfum

1) préparation du pré-polymère de silicone :

Dans un bécher est mélangé à l'abri de l'oxygène et de l'humidité (circulation d'azote sec) :

- 0,1 mole de HDMI (hexaméthylène diisocyanate), soit 16,8 g
- 0, 2 mole de MTMO (gamma-mercaptopropyl triméthoxysilane), soit 39,8 g.

Le tout est mélangé et porté 10h à 60°C puis refroidi à température ambiante (20°C à 25 °C).

2) Encapsulation

111 g de parfum « Blue wave d'expressions parfumées » est mélangé de façon à obtenir une solution transparente à 8,5 g du pré-polymère de silicone préparé précédemment en 1) et à 14,8 g de polysilicate d'éthyle TES 40 de Wacker. Ce mélange constituera la phase lipophile interne des microcapsules.

Dans un bécher de 500 $cm^3$ maintenu à 45°C on introduit sous agitation :

- 130 g d'eau du robinet
- 1 g d' hydroxyéthylcellulose (250M de Aqualon)
- 2 g de Lupasol PA 140 (BASF)
- 2,7 g de mélanine en poudre
- 5,95 g de glyoxal à 40%
- 1,16 g de glutaraldéhyde à 50%
- et 6 g d'acide nitrique à 20%.

L'agitateur est équipé d'une hélice à 5 pales droites d'un diamètre de 6 cm. La vitesse d'agitation est augmentée jusqu'à 1600 tr/min puis le mélange préparé précédemment à partir du parfum « Blue wave » est émulsionné dans le mélange aqueux précédent. Le pH est alors de 3,8.

La température est maintenue à 45°C pendant 2h puis portée à 50°C pendant 1h, période durant laquelle la vitesse d'agitation est réglée à 1800 tr/min de façon à obtenir un diamètre moyen de 10 µm, puis elle est diminuée à 1300 tr/min.

0,5 g de Fixapret NF (BASF) sont alors ajoutés. La température est maintenue à 50°C pendant 1h, puis portée à 80°C pendant 6h pour effectuer la polymérisation complète des 2 couches de polymères de l'enveloppe des microcapsules. L'émulsion est ensuite refroidie à 30°C.

Puis le pH est remonté à 7,0 lentement avec de la lessive de potasse.

Résultats : les microcapsules obtenues présentent des performances d'étanchéité comparables aux microcapsules mélamine/formol standard mais présentent un taux de formaldéhyde de 0 ppm et une tenue aux tensio-actifs supérieure.

**Exemple comparatif 6 :** performances des microcapsules dans une lessive liquide L'objectif est d'incorporer des microcapsules à une lessive liquide (*Fabric washing liquid* HC 0097/12), et d'évaluer la perméabilité des microcapsules dans ce milieu liquide.

**Description des capsules testées**

Quatre types de microcapsules avec le même parfum « Blue » sont testés :

S : Microcapsules de silicone, préparées selon l'exemple 3 du brevet français FR 2937248 ;

M : microcapsules mélamine formaldéhyde réalisées selon le brevet US 4406816 exemple 1 (avec parfum) ;

G : microcapsules de gélatine réalisées selon le brevet EP 0 674 942 B1 Exemple 1 (avec parfum) ;

SM : microcapsules à deux couches selon la présente invention de l'exemple 3 ci-dessus.

Les caractéristiques des microcapsules préparées sont regroupées dans le tableau 1 ci-après :

Tableau 1

| Type | Référence interne | Diamètre moyen | Formaldéhyde ppm |
|---|---|---|---|
| **S** (comparatif) | 4331 | 6 μm | 0 |
| **SM** (invention) | 4643 | 6,5 μm | 100 |
| **M** (comparatif) | 4623 | 6,8 μm | 450 |
| **G** (comparatif) | 4624 | 7 μm | 5 |

**Mode opératoire**

Les microcapsules à 35% en poids d'actif sont incorporées à la lessive dans un rapport pondéral 5/95 et mélangées à l'aide d'une spatule,

Chacun des mélanges est observé à l'oeil nu puis au microscope et sa stabilité est suivie dans le temps.

**Résultats**

Après observation au microscope et prise de photographie, les observations relatives aux différents mélanges, aussitôt après l'incorporation à la lessive, sont regroupées dans le tableau 2 ci-après :

Tableau 2

| Microcapsules | Dans le « Fabric washing liquid » |
|---|---|
| S 4331 | Bien dispersées mais un peu déformées |
| SM 4643 | Bien dispersées mais quelques capsules se collent / Quelques petits agglomérats |
| M 4623 | Bien dispersées / Rare agglomérats |
| G 4624 | Bien dispersées |

Une semaine après un vieillissement accéléré à 40°C, on observe à nouveau les mélanges et on évalue l'intensité olfactive : plus celle-ci est importante, plus les microcapsules ont souffert (voir tableau 3 ci-dessous) :

Tableau 3

| Microcapsules | Aspect de la lessive | Observation au microscope | Intensité du parfum |
|---|---|---|---|
| S 4331 | Tache blanche / Très épais / Couleur beige | Bien dispersées mais cabossées | Odeur forte |
| SM 4643 | Couleur blanc beige bien homogène | Assez bien dispersées | Odeur très faible Difficilement perceptible |
| M 4623 | Homogène, tache blanche sur le dessus / Moins épais / Couleur blanc beige | Bien dispersées | Odeur relativement faible, Très perceptible |
| G 4624 | Couleur blanc beige bien homogène | Bien dispersées | Odeur très forte |

On effectue alors le classement présenté ci-après, de l'échantillon dégageant le moins de parfum à celui qui sent le plus fort dans le bécher. Les microcapsules les plus étanches ayant le meilleur rendu olfactif correspondent à l'essai qui sent le moins fort, comme schématisé sur l'échelle ci-après :

*Intensité olfactive faible* → *Intensité olfactive forte*

*Microcapsules restant étanches* — *Microcapsules devenues poreuses*

SM 4643 < M 4623 < S 4331 < G 4624

**Conclusion**

Les microcapsules SM à double paroi sont les plus étanches, elles relarguent moins de parfum que les autres microcapsules car elles ont été moins attaquées par les tensio-actifs de la lessive liquide. Les microcapsules "mélamine" M sont moyennement étanches, elles souffrent en plus d'un taux de formaldéhyde très élevé. Les microcapsules "silicone" S sont peu résistantes dans les solutions de tensio-actifs. Quant aux microcapsules "gélatine" G, se sont celles qui dégagent le plus d'odeur et qui sont donc devenues les plus poreuses.

**Revendications**

**1.** Procédé de fabrication de microcapsules à réservoir, du type renfermant un principe actif dans une enveloppe à base de polymère comprenant les étapes consistant à:

(i) disperser au moins un principe actif lipophile dans une phase continue aqueuse, de manière à former une émulsion ou une dispersion de gouttelettes de type huile dans l'eau,
(ii) polymériser in situ au moins un précurseur du polymère à la périphérie desdites gouttelettes pour former la paroi de l'enveloppe des microcapsules, en enfermant le principe actif,

**caractérisé en ce que**
l'étape de polymérisation (ii) est précédée de l'introduction dans la phase lipophile d'un ou plusieurs composés A portant des groupes alcoxysilanes et l'introduction dans la phase aqueuse de monomères organiques aminés B comportant au moins un groupe choisi parmi les groupes mélamine, urée, glycoluril, benzoguanamine, ou dicyandiamide et d'un ou de plusieurs aldéhydes, et/ou de leurs pré-polymères,
les composés A et B étant ensuite, de préférence simultanément, respectivement hydrolysés et condensés en milieu acide en un polymère silicone et un polymère organique aminé, liés entre eux par des liaisons polaires, hydrogène ou covalentes, composant la paroi de l'enveloppe des microcapsules.

**2.** Procédé selon la revendication 1, **caractérisé en ce que** les deux polymères silicone et organique aminé sont formés simultanément par catalyse acide à un pH compris entre 2 et 6.

**3.** Procédé selon l'une des revendications 1 ou 2, **caractérisé en ce que** les deux polymères silicone et organique aminé sont formés simultanément par catalyse acide à un pH situé entre 3 et 5, par ajout dans l'émulsion ou dispersion huile dans l'eau d'au moins un acide comprenant l'acide nitrique.

**4.** Procédé selon l'une des revendications 1 à 3, **caractérisé en ce que** le pré-polymère organique aminé est une résine mélamine-formaldéhyde et/ou urée-formaldéhyde.

**5.** Procédé selon l'une des revendications 1 à 3, **caractérisé en ce que** l'aldéhyde utilisé pour fabriquer le pré-polymère organique aminé est choisi parmi l'acétaldéhyde, le glyoxal, le glutaraldéhyde, ou un mélange de ceux-ci, et/ou un ou plusieurs acétals de ces aldéhydes.

**6.** Procédé selon l'une des revendications précédentes, **caractérisé en ce que** le polymère organique aminé est copolymérisé avec des monomères hydroxylés aliphatiques ou aromatiques et/ou des aldéhydes aromatiques.

**7.** Procédé selon l'une des revendications précédentes, **caractérisé en ce que** le ou les composés A portant des fonctions alcoxysilanes est/sont choisi(s) parmi les composés de formule (I) ou (II) ci-après :

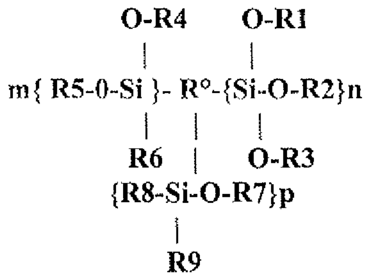 et/ou 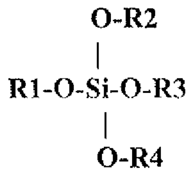

(I) (II)

dans lesquels R1, R2, R3, R4, R5, R6, R7, R8, R9 sont des radicaux alkyle, linéaires ou cycliques, substitués ou non,
R° est une molécule organique et/ou silicone,
les groupes entre {} étant reliés à R° par un atome de silicium et sont présents m, n, ou p fois, et m, n, p pouvant être nuls individuellement, mais la somme m+n+p étant au moins égale à 1.

**8.** Procédé selon la revendication 7, **caractérisé en ce que** le composé de formule (II) est choisi parmi le polysilicate de méthyle, le polysilicate d'éthyle ou un mélange de ceux-ci.

**9.** Procédé selon l'une des revendications précédentes, **caractérisé en ce que** le polymère de silicone représente de 5 % à 95 %, de préférence de 10 à 90%, de préférence encore de 15 à 85 %, du poids total des polymères formant la paroi de la microcapsule.

**10.** Microcapsules préparées au moyen du procédé selon l'une quelconque des revendications précédentes, comprenant un polymère silicone et un polymère organique aminé, liés entre eux par des liaisons polaires, hydrogène ou covalentes, composant la paroi de l'enveloppe des microcapsules.

**11.** Microcapsules selon la revendication 10, renfermant en tant que principe actif une molécule odorante, tel qu'un parfum.

**12.** Utilisation des microcapsules préparées au moyen du procédé selon l'une quelconque des revendications 1 à 9 dans des formulations contenant des tensio-actifs.

**13.** Utilisation des microcapsules préparées au moyen du procédé selon l'une quelconque des revendications 1 à 9 dans des lessives liquides, des lessives en poudre ou des détergents ménagers et industriels.

**14.** Utilisation des microcapsules préparées au moyen du procédé selon l'une quelconque des revendications 1 à 9 dans des adoucissants pour textiles.

**15.** Utilisation des microcapsules préparées au moyen du procédé selon l'une quelconque des revendications 1 à 9 dans des shampoings, des produits de conditionnement pour cheveux, des dentifrices, des savons liquides, des nettoyants corporels ou des lotions.

## Patentansprüche

**1.** Verfahren zur Herstellung von Mikrokapseln mit Reservoir des Typs, einen Aktivstoff in einer Hülle auf der Basis von Polymer einschließend, das die folgenden Schritte umfasst:

(i) Verteilen mindestens eines lipophilen Aktivstoffs in einer kontinuierlichen Wasserphase derart, dass eine Emulsion oder eine Tröpfchendispersion vom Typ Öl in Wasser gebildet wird,
(ii) Polymerisieren in situ mindestens eines Vorläufers des Polymers an der Peripherie der Tröpfchen, um die Wand der Hülle der Mikrokapseln zu bilden, bei Einschluss des Aktivstoffs,

**dadurch gekennzeichnet, dass**
vor dem Polymerisationsschritt (ii) das Einleiten in die lipophile Phase einer oder mehrerer Verbindungen A erfolgt,

die Alcoxysilangruppen tragen, und das Einleiten in die wässrige Phase von organischen Aminmonomeren B erfolgt, die mindestens eine Gruppe aufweisen, die aus den Gruppen Melamin, Urea, Glycoluril, Benzoguanamin oder Dicyandiamid ausgewählt ist, und eines oder mehrerer Aldehyde und/oder von ihren Pre-Polymere,
wobei die Verbindungen A und B danach, vorzugsweise gleichzeitig, jeweils in saurem Milieu in ein Silikonpolymer und ein organisches Aminpolymer hydrolysiert und kondensiert werden, die miteinander mittels polarer, Wasserstoff- oder kovalenter Bindungen verbunden sind, die die Wand der Hülle der Mikrokapseln bilden.

2. Verfahren nach Anspruch 1, **dadurch gekennzeichnet, dass** die zwei Polymere, das Silikonpolymer und das organische Aminpolymer, durch saure Katalyse bei einem pH zwischen 2 und 6 gleichzeitig gebildet werden.

3. Verfahren nach einem der Ansprüche 1 oder 2, **dadurch gekennzeichnet, dass** die zwei Polymere, das Silikonpolymer und das organische Aminpolymer, durch Hinzufügen in die Öl-in-Wasser-Emulsion oder -Dispersion von mindestens einer Säure, die Salpetersäure umfasst, durch saure Katalyse bei einem pH zwischen 3 und 5 gleichzeitig gebildet werden.

4. Verfahren nach einem der Ansprüche 1 bis 3, **dadurch gekennzeichnet, dass** das organische Pre-Aminpolymer ein Melaminformaldehyd- und/oder Urea-Formaldehydharz ist.

5. Verfahren nach einem der Ansprüche 1 bis 3, **dadurch gekennzeichnet, dass** das Aldehyd, das für die Herstellung des organischen Pre-Aminpolymers verwendet wird, aus dem Acetaldehyd, dem Glyoxal, dem Glutaraldehyd oder einem Gemisch derselben und/oder einem oder mehreren Acetalen dieser Aldehyde ausgewählt ist.

6. Verfahren nach einem der vorangehenden Ansprüche, **dadurch gekennzeichnet, dass** das organische Aminpolymer mit aliphatischen oder aromatischen hydroxylierten Monomeren und/oder aromatischen Aldehyden copolymerisiert wird.

7. Verfahren nach einem der vorangehenden Ansprüche, **dadurch gekennzeichnet, dass** die Verbindung(en) A, die Alcoxysilangruppen trägt/tragen, aus den Verbindungen der nachfolgenden Formel (I) oder (II) ausgewählt ist/sind:

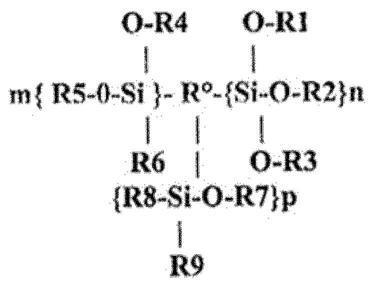

(I)

und/oder

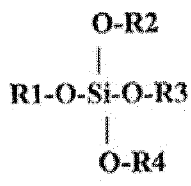

(II)

wobei R1, R2, R3, R4, R5, R6, R7, R8, R9 Alkylradikale, lineare oder cyclische, substitutiert oder nicht, sind,
R° ein organisches und/oder Silikonmolekül ist,
die Gruppen zwischen {} an R° mit einem Silikonatom gebunden sind und m, n oder p Mal vorhanden sind, und wobei m, n, p einzeln Null sein können, aber die Summe m+n+p mindestens gleich 1 ist.

8. Verfahren nach Anspruch 7, **dadurch gekennzeichnet, dass** die Verbindung der Formel (II) aus dem Methylpolysilikat, dem Ethylpolysilikat oder einem Gemisch derselben ausgewählt ist.

9. Verfahren nach einem der vorangehenden Ansprüche, **dadurch gekennzeichnet, dass** das Silikonpolymer 5 % bis 95 %, vorzugsweise 10 bis 90%, auch vorzugsweise 15 bis 85 % des Gesamtgewichts der Polymere darstellt, die die Wand der Mikrokapsel bilden.

10. Mikrokapseln, hergestellt mittels des Verfahrens nach einem der vorangehenden Ansprüche, umfassen ein Silikonpolymer und ein organisches Aminpolymer, die miteinander mittels polarer, Wasserstoff- oder kovalenter Bindungen verbunden sind, die die Wand der Hülle der Mikrokapseln bilden.

**11.** Mikrokapseln nach Anspruch 10, welche als Aktivstoffe ein Duftmolekül wie einen Duftstoff einschließen.

**12.** Verwendung von Mikrokapseln, hergestellt mittels des Verfahrens nach einem der Ansprüche 1 bis 9, in Formulierungen, welche Tenside enthalten.

**13.** Verwendung der Mikrokapseln, hergestellt mittels des Verfahrens nach einem der Ansprüche 1 bis 9, in Flüssigwaschmitteln, Pulverwaschmitteln oder Haushalts- und Industriereinigern.

**14.** Verwendung der Mikrokapseln, hergestellt mittels des Verfahrens nach einem der Ansprüche 1 bis 9, in Textilweichspülern.

**15.** Verwendung der Mikrokapseln, hergestellt mittels des Verfahrens nach einem der Ansprüche 1 bis 9, in Shampoons, Haarconditionern, Zahncremes, Flüssigseifen, Körperwaschmitteln oder Lotionen.

## Claims

**1.** A method for manufacturing microcapsules with a reservoir, of the type containing an active ingredient in a polymer-based casing comprising the steps consisting of:

(i) dispersing at least one lipophilic active ingredient in an aqueous continuous phrase, so as to form an emulsion or a dispersion of droplets of the oil-in-water type,
(ii) polymerizing in situ at least one precursor of the polymer at the periphery of said droplets in order to form the wall of the casing of the microcapsules, while containing the active ingredient,

**characterized in that**
the polymerization step (ii) is preceded with the introduction into the lipophilic phase of one or several compounds A bearing alkoxysilane groups and with the introduction into the aqueous phase of organic aminated monomers B including at least one group selected from among melamine, urea, glycoluril, benzoguanamine, or dicyandiamide groups and from one or several aldehydes, and/or their pre-polymers,
the compounds A and B being then, preferably simultaneously, respectively hydrolyzed and condensed in an acid medium into a silicone polymer and an aminated organic polymer, bound together through polar, hydrogen or covalent bonds making up the wall of the casing of the microcapsules.

**2.** The method according to claim 1, **characterized in that** both silicone and aminated organic polymers are simultaneously formed by acid catalysis at a pH comprised between 2 and 6.

**3.** The method according to one of claims 1 or 2, **characterized in that** both silicone and aminated organic polymers are simultaneously formed by acid catalysis at a pH located between 3 and 5, by adding into the oil-in-water emulsion or dispersion at least one acid comprising nitric acid.

**4.** The method according to one of claims 1 to 3, **characterized in that** the aminated organic pre-polymer is a melamine/urea formaldehyde and/or a urea/formaldehyde resin.

**5.** The method according to one of claims 1 to 3, **characterized in that** the aldehyde used for making the aminated organic pre-polymer is selected from among acetaldehyde, glyoxal, glutaraldehyde, or a mixture thereof, and/or one or several acetals of these aldehydes.

**6.** The method according to one of the preceding claims, **characterized in that** the aminated organic polymer is copolymerized with aliphatic or aromatic hydroxylated monomers and/or with aromatic aldehydes.

**7.** The method according to one of the preceding claims, **characterized in that** the compound(s) A bearing alkoxysilane functions is/are selected from among the compounds of formula (I) or (II) hereafter:

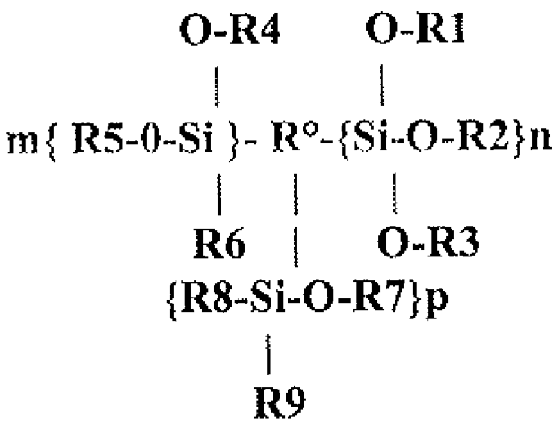 and/or 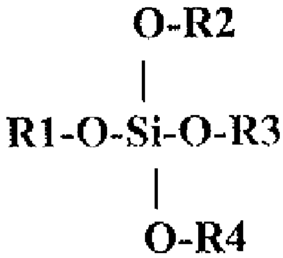

(I) (II)

wherein R1, R2, R3, R4, R5, R6, R7, R8, R9 are cyclic or linear alkyl radicals, either substituted or not,
R° is an organic molecule or silicone,
the groups between {} being bound with R° through a silicon atom and are present m, n or p times, and m, n, p may be individually zero but the sum m+n+p being at least equal to 1.

8. The method according to claim 7, **characterized in that** the compound of formula (II) is selected from among poly(methyl silicate), poly(ethyl silicate) or a mixture thereof.

9. The method according to one of the preceding claims, **characterized in that** the silicone polymer represents from 5% to 95%, preferably from 10 to 90%, still preferably from 15 to 85%, based on the total weight of the polymers forming the wall of the microcapsule.

10. Microcapsules prepared by means of the method according to any of the preceding claims, comprising a silicone polymer and an aminated organic polymer, bound together with polar, hydrogen or covalent bonds, making up the casing of the microcapsules.

11. The microcapsules according to claim 10, containing as an active ingredient an odorous molecule such as a perfume.

12. The use of microcapsules prepared by means of the method according to any of claims 1 to 9, in formulations containing surfactants.

13. The use of microcapsules prepared by means of the method according to any of claims 1 to 9, in liquid detergents, powder detergents or home and industrial detergents.

14. The use of microcapsules prepared by means of the method according to any of claims 1 to 9, in softeners for textiles.

15. The use of microcapsules prepared by means of the method according to any of claims 1 to 9, in shampoos, hair conditioning products, toothpastes, liquid soaps, body cleansers or lotions.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4406816 A
- US 4824823 A
- US 4396670 A
- EP 1797947 A
- US 7968510 B
- US 20100009893 A
- US 20100247941 A
- WO 2011161618 A
- FR 2937248
- FR 2913887
- EP 0674942 B1